# EUROPÄISCHE PATENTANMELDUNG

(11) **EP 2 366 388 A1**
(43) Veröffentlichungstag der Anmeldung: **21.09.2011**
(21) Anmeldenummer: 10002806.7
(22) Anmeldetag: 17.03.2010
(51) Int. Cl.: A61K 9/70, A61K 31/485

(54) **Nicht-okklusives transdermales therapeutisches System zur Verabreichung von Buprenorphin**

(71) Anmelder: Ratiopharm GmbH, 89079 Ulm (DE)
(72) Erfinder: Becker, Ulrich, Dr., 40764 Langenfeld (DE); Breitenbach, Armin, Dr., 51371 Leverkusen (DE)

(57) **Zusammenfassung**

Die vorliegende Erfindung betrifft ein nicht-okklusives transdermales therapeutisches System zur Verabreichung von Buprenorphin, ein Verfahren zur Herstellung des Systems und die Verwendung einer nicht-okklusiven Schicht als Trägerschicht für ein entsprechendes System.

## Beschreibung

Die vorliegende Erfindung betrifft ein nicht-okklusives transdermales therapeutisches System zur Verabreichung von Buprenorphin, ein Verfahren zur Herstellung des Systems und die Verwendung einer nicht-okklusiven Schicht als Trägerschicht für ein entsprechendes System.

Buprenorphin ist ein starkes Opioid. Seine Wirksamkeit bei der Behandlung starker Schmerzen, insbesondere Tumorschmerzen, ist bekannt. Hierbei zeigt es eine im Vergleich zu Morphin um etwa zwei Größenordnungen höhere Potenz.

Die Weltgesundheitsorganisation (WHO) hat zur Schmerztherapie (insbesondere für die Tumorschmerztherapie) ein Stufenschema entwickelt. Dieses umfasst drei Stufen. Bei der Schmerztherapie wird mit Stufe 1 begonnen, wobei bei ungenügender Wirksamkeit zur nächsthöheren Stufe übergegangen wird. Stufe 1 umfasst die Schmerztherapie mittels eines nicht-opioiden Analgetikums, optional in Kombination mit einem anderen Medikament als Adjuvans. Stufe 2 umfasst die Gabe eines schwachen Opioids, optional in Kombination mit einem nicht opioiden Analgetikum und/oder Adjuvans. Die Schmerztherapie entsprechend Stufe 3 beinhaltet die Gabe eines starken Opioids, optional in Kombination mit einem nicht-opioiden Analgetikum und/oder einem Adjuvans. So kann auch bei Tumorschmerzen Schmerzfreiheit erzielt werden.

Die WHO empfiehlt, dass die Schmerzmittel in bestimmten Zeitabständen gegeben werden und nicht nur bei Bedarf. Laut WHO ist dieser 3-Schritt-Ansatz, das richtige Medikament in der richtigen Dosis zur richtigen Zeit zu verabreichen, kostengünstig und 80 bis 90 Prozent effektiv.

Zur Eignung eines Medikaments bei der Schmerztherapie gemäß Stufe 3 des WHO-Stufenschemas ist es also nicht nur erforderlich, ein hinreichend starkes Opioid bereitzustellen, sondern es ist ebenso erforderlich, dass es dem Körper in hinreichend großen Dosen verfügbar gemacht wird. Wünschenswert sind auch, eine gute Verträglichkeit bei dauerhafter Anwendung und ein verhältnismäßig gleichmäßiger Fluss von Opioid, so dass eine verhältnismäßig gleich bleibende therapeutische Wirkung gewährleistet werden kann.

Die Eignung von Buprenorphin zur Behandlung chronischer Schmerzen und insbesondere Tumorschmerzen hat zur Entwicklung von Systemen geführt, mit denen Buprenorphin einem Patienten über einen längeren Zeitraum (mehrere Tage) in einer etwa gleich bleibenden Dosis zugeführt werden kann. Dazu sind transdermale therapeutische Systeme (TTS) für Buprenorphin, z.B. in Form wirkstoffhaltiger Pflaster, im Stand der Technik bekannt. Problematisch dabei ist jedoch die schlechte Hautgängigkeit von Buprenorphin.

In EP 0 819 438 A2 wird darauf hingewiesen, dass die transdermale Absorption von Buprenorphin und seinen Salzen schlecht ist, so dass der Wirkstoff kaum in der benötigten Dosis über eine in der Praxis verfügbare Fläche von etwa 100 cm² oder weniger durch die Haut absorbiert werden können. Diese Erkenntnis ist für den Fachmann nicht überraschend, da diese Schwierigkeit nicht nur bei Buprenorphin, sondern auch bei vielen anderen stark lipophilen Substanzen beobachtet wird.

Eine der Möglichkeiten, diesem Problem zu begegnen, ist es, sich dem Effekt der Okklusion zu Nutze zu machen. Hierbei wird ein wasserdampfundurchlässiges System eingesetzt, das zu einem Wasserdampfstau, einer Quellung und Auflockerung der Strukturen in den oberen Schichten der Haut führt. Hierdurch wird die transdermale Aufnahme von Wirkstoffen erleichtert. Ein üblicher Weg, um Okklusion zu erzeugen, liegt in der Verwendung wasserdampfundurchlässiger Träger- oder Rückschichten. Dementsprechend schlägt die EP 0 819 438 A2 als Rückschichten Filme, die aus verschiedenen Kunststoffen bestehen können, Metallfolien, Laminatfilme daraus oder auch Laminate von solchen nicht-porösen Filmen mit gewebten oder nicht-gewebten Stoffen vor. Rückschichten, die keine Okklusion hervorrufen würden, sind im EP 0 819 438 A2 nicht offenbart.

Auch aus EP 0 432 945 A1 ist ein transdermales therapeutisches System enthaltend Buprenorphin bekannt. Es wird auf die Lipophilie von Buprenorphin verwiesen. Davon ausgehend wird gelehrt, dass die Rückschichten der TTS impermeable Filme sind, also wasserundurchlässig sind.

Ebenso offenbart WO 91/19474 A1 die transdermale Verabreichung von Buprenorphin. Geeignete transdermale therapeutische Systeme enthalten gemäß WO 91/19474 A1 eine Rückschicht aus einer einzelnen oder mehreren Schichten. Die Rückschichten können beispielsweise aus einem elastomeren Polymer bestehen, wobei es zur Sicherstellung der Okklusion nötig sein kann, eine zusätzliche Schicht eines okklusiven (eine Okklusion hervorrufenden) Materials vorzusehen. Alternativ können nicht-elastomere okklusive Polymere für die Rückschicht verwendet werden.

WO 01/91718 A2 beschreibt ganz allgemein, dass die Verabreichung von Wirkstoffen durch die Haut in der Mehrzahl der Fälle durch eine geringe Durchlässigkeit der Haut erschwert wird, dass ihre Aufnahme durch Okklusion begünstigt wird und deshalb die Verwendung wasserdampfundurchlässiger Rückschichten bei fast allen auf dem Markt befindlichen transdermalen therapeutischen Systemen anzutreffen ist. Weiter offenbart WO 01/91718 A2, dass als wasserdampfundurchlässige Schichten in der Regel Folien aus Polyethylenterephthalat (PET) verwendet werden, was jedoch zu einem geringen Tragekomfort führt, da diese Folien meist unelastisch und starr sind, sich der Haut wenig anzuschmiegen vermögen und Dehnungen und Stauchungen der beklebten Hautoberfläche nicht in physiologischer Weise zulassen. Ein weiterer Nachteil, auf den WO 01/91718 A2 verweist ist, dass bei einer solchen starren Rückschicht die Tragedauer eines transdermalen therapeutischen Systems dadurch verkürzt wird, dass die andauernden mechanischen Spannungen von der Klebeschicht nicht dauerhaft gehalten werden können. WO 01/91718 A2 verweist weiter darauf, dass in dieser Hinsicht vorteilhaftere elastische Folien hingegen gut wasserdampfdurchlässig sind und eine nur geringe Okklusion, wenn überhaupt, erzeugen, und das Gewebe oder Vliesstoffe als Rückschichten praktisch keine Okklusion erzeugen, die für die Durchlässigkeit der Haut von Bedeutung sein könnte. Als Lösung, schlägt die WO 01/91718 A2 vor, eine andere Schicht wasserdampfsperrend auszubilden.

Das allgemeine Problem der kritischen Hautgängigkeit (Permeation) einiger Wirkstoffe und dessen Lösung durch okklusive Rückschichten wird auch in US 5,006,342 angesprochen. Darin wird ein Vergleich der in vitro-Hautpermeation des (wie das Opiod Buprenorphin ebenfalls) stark lipophilen Opioids Fentanyl zwischen transdermalen therapeutischen Systemen mit einer okklusiven bzw. einer nicht-okklusiven Schicht beschrieben. Die Permeation unter Verwendung des okklusiven Systems war zehnmal größer als die unter Verwendung des nicht-okklusiven Systems.

Ein bekanntes TTS enthaltend Buprenorphin ist Transtec PRO® von Grünenthal. Es ist ein Matrixpflaster (Wirkstoff in einer Adhäsivschicht), für eine Tragedauer von 4 Tagen ausgelegt und in drei verschiedenen Ausführungen mit nominalen Abgaberaten von 35, 52,5 und 70 µg/h erhältlich. Die Gesamtfläche ist in der 35 µg/h-Ausführung 50 cm², die wirkstoffabgebende Fläche ist dort 25 cm². Die Gesamtfläche ist im Vergleich zur wirkstoffabgebenden Fläche hoch, da eine zusätzliche klebende Schicht erforderlich ist. Dieses System hat einen verhältnismäßig aufwändigen Aufbau aus einer entfernbaren Schutzschicht, daran anschließend einer Adhäsivschicht enthaltend Buprenorphin, einer PET-Folie, einer weiteren klebenden Schicht, die kein Buprenorphin enthält, und abschließend einem rückseitigen Abdeckgewebe. Es handelt sich also um ein System mit einer wasserdampfsperrenden Schicht, die eine Okklusion hervorruft.

Ein weiteres Marktprodukt wird von AWD/Acino hergestellt, das sog. "Buprenorphin AWD® Matrix Transdermale Pflaster". Es umfasst ebenfalls eine wasserdampfsperrende Schicht. Die Hautpermeation wird zudem durch weitere permeationsfördende Substanzen, wie in EP 1 660 055 beschrieben, erhöht.

Der Fachmann entnimmt somit dem Stand der Technik, dass zur effektiven Verabreichung von Buprenorphin mittels eines transdermalen therapeutischen Systems die Ausnutzung der Okklusion unbedingt erforderlich wird. Die die Okklusion ausnützenden wasserdampfsperrenden Systeme haben jedoch, wie zum Teil bereits angedeutet, eine Reihe von Nachteilen: die oftmals eingesetzten starren und unelastischen Polymerfolien oder Metallfolien führen zu geringem Tragekomfort und einer verkürzten Tragedauer; es kommt zu Feuchtigkeitsstau und oft zu Hautirritationen; oft ist eine zufriedenstellende Haftung des transdermalen therapeutischen Systems auf der Haut nicht gewährleistet; und es handelt sich zum Teil um recht aufwändige Systeme.

Die Aufgabe der vorliegenden Erfindung ist somit, ein effektives transdermales therapeutisches System zur Verabreichung von Buprenorphin bereitzustellen, mit dem einer oder mehrere der genannten Nachteile überwunden oder zumindest verringert werden. Zudem sind transdermale therapeutische Systeme wünschenswert, die Vorteile hinsichtlich Lagerstabilität, mechanischer Beständigkeit, Einfachkeit der Herstellung, Wirtschaftlichkeit, problemloser Wiederentfernbarkeit (z.B. schmerz- und rückstandsfrei) und ihrer Sicherheit, etwa hinsichtlich eines geringen Restgehalts an Buprenorphin, zeigen.

Es hat sich entgegen den Annahmen des Stands der Technik und daher überraschend gezeigt, dass diese Aufgabe gelöst wird durch ein transdermales therapeutisches System umfassend eine Trägerschicht und eine Adhäsivschicht, die Buprenorphin und/oder eines seiner pharmazeutisch annehmbaren Salze enthält, und optional eine ablösbare Schutzschicht, wobei das transdermale therapeutische System nicht-okklusiv ist und zur Schmerztherapie entsprechend Stufe 3 des dreistufigen WHO-Stufenschemas geeignet ist. Das erfindungsgemäße System kann auch nur aus den genannten Schichten bestehen.

"Nicht-okklusiv" bedeutet im Zusammenhang mit dem erfindungsgemäßen transdermalen therapeutischen System, dass das transdermale therapeutische System, abgesehen von der gegebenenfalls vorhandenen Schutzschicht, frei von okklusiven Schichten ist. Unter okklusiven Schichten werden solche Schichten verstanden, die die Diffusion von Wasserdampf verhindern oder stark einschränken, d.h. eine nach DIN 53122 gemessene Wasserdampfdurchlässigkeit von kleiner als 150 g/(m^{2*}24 h), bevorzugt kleiner als 100 g/(m^{2*}24 h), besonders bevorzugt kleiner als 60 g/(m^{2*}24 h) und insbesondere kleiner als 20 g/(m^{2*}24 h) haben. Eine nicht-okklusive Schicht ist dementsprechend eine Schicht, die nicht dieser Definition einer okklusiven Schicht entspricht.

Mit "Adhäsivschicht" sowie mit "Kleberschicht" werden Schichten bezeichnet, die sich durch Adhäsionswirkung, also eine Klebewirkung auszeichnen. Diese Schichten enthalten eine oder mehrere adhäsive Substanzen (Klebstoffe) und ermöglichen es, durch ihren Kontakt mit der Haut, eine Haftung des erfindungsgemäßen transdermalen therapeutischen Systems an der Haut eines Patienten zu vermitteln. Der Patient ist vorzugsweise ein Mensch. Die Adhäsivschicht und die Kleberschicht sind vorzugsweise Schichten, die für ihre mechanische Integrität (z.B. mechanische Beständigkeit) eines Kontakts mit einem anderen Material (z.B. der Trägerschicht oder einer anderen Schicht) bedürfen. Wie erwähnt, enthält die Adhäsivschicht den Wirkstoff des Buprenorphins und/oder eines seiner pharmazeutisch annehmbaren Salze. Diese ist darin verteilt, vorzugsweise gleichmäßig. Die gleichmäßige Verteilung ist vorzugsweise eine Dispersion oder eine Lösung. Die Adhäsivschicht bestimmt vorzugsweise wesentlich, insbesondere allein, die kontrollierte Abgabe (z.B. die Abgaberate) des Buprenorphins bzw. seinen Salzen aus dem erfindungsgemäßen System.

"Trägerschicht" bezeichnet erfindungsgemäß eine Schicht, die wesentlich zur mechanischen Integrität (z.B. mechanischen Beständigkeit und/oder Festigkeit) des erfindungsgemäßen transdermalen therapeutischen Systems beiträgt, insbesondere dafür verantwortlich ist. Vorzugsweise ist die Trägerschicht frei von Buprenorphin und seinen Salzen. Sie entfaltet eine mechanische und/oder chemische Schutzwirkung für andere Schichten, insbesondere die Adhäsivschicht, z.B. vor Beschädigung oder unerwünschten Substanzen aus der Umgebung. Weiterhin unterstützt die Trägerschicht die gerichtete Abgabe von Buprenorphin an die Haut.

Die erfindungsgemäß gegebenenfalls vorhandene "ablösbare Schutzschicht" ist eine Schicht, die vor dem Aufbringen des erfindungsgemäßen Systems auf die Haut entfernt wird. Sie ist vor dem Ablösen in Kontakt mit einer Schicht mit Adhäsionswirkung (Adhäsivschicht oder Kleberschicht) und schützt diese vor Kontakt mit anderen Oberflächen als der gewünschten Hautstelle. Vorzugsweise schützt sie sie auch mechanisch und/oder chemisch, z.B. vor Beschädigung oder unerwünschten Substanzen aus der Umgebung. Zudem dient sie dem Erhalt der Klebeeigenschaften der Klebeschicht während der Lagerung. Die ablösbare Schutzschicht ist daran angepasst, einfach und rückstandsfrei von der Schicht mit Adhäsionswirkung abgelöst zu werden, vorzugsweise durch Abziehen. Vorzugsweise ist die ablösbare Schutzschicht so ausgeführt, dass kein Buprenorphin oder Salz von Buprenorphin sie durchdringen kann.

An den Stellen der vorliegenden Anmeldung, an denen zwischen Buprenorphin und Salzen von Buprenorphin differenziert wird, ist mit ersterem Buprenorphin-Base gemeint. Stellen, an denen nicht differenziert wird und allgemein von Buprenorphin die Rede ist, beziehen sich auf sowohl auf Buprenorphin-Base als auch auf Salze von Buprenorphin.

Die erwähnten Schichten des erfindungsgemäßen Systems haben erfindungsgemäß vorzugsweise eine im Wesentlichen gleich bleibende Dicke, die z.B. an der dicksten Stelle um nicht mehr als 20%, 10% oder 5% größer ist als an der dünnsten Stelle. Sie sind vorzugsweise hautverträglich und nicht-toxisch. Vorzugsweise sind sie inert gegenüber Buprenorphin und/oder anderen Inhaltsstoffen des erfindungsgemäßen Systems.

Im auf die Haut aufgeklebten Zustand lässt das erfindungsgemäße nicht-okklusive transdermale therapeutische System (im Folgenden manchmal nur System genannt) von der Haut abgegebenen Wasserdampf - zumindest in gewissem Ausmaß - passieren. Dadurch, dass von okklusiven Schichten abgesehen wird, erlaubt das erfindungsgemäße transdermale therapeutische System, verbesserte Eigenschaften hinsichtlich des Tragekomforts, des Feuchtigkeitsstaus, der Hautirritation, der Haftung, der Tragedauer, der geringeren Starrheit und höheren Elastizität des Systems und/oder weitere oben und im Folgenden diskutierte Vorteile zu erzielen.

Der Aufbau des erfindungsgemäßen Systems ohne okklusive Schichten ermöglicht es, starre und unelastische Schichten wie Polymerfolien und Metallfolien zu vermeiden, womit ein entsprechendes System die Bewegungen der Haut besser nachvollziehen kann. Dies kann den, Tragekomfort verbessern und die Tragedauer verlängern, da die physiologischen Dehnungen und Stauchungen der beklebten Hautoberfläche nicht verhindert werden und es zu geringeren Spannungen kommt. Der nicht-okklusive Aufbau kann auch den Feuchtigkeitsstau verringern oder beseitigen, der vom Patienten als unangenehm empfunden werden kann. Auch Hautirritationen wie z.B. Hautrötung oder Juckreiz können besser vermieden werden. Das erfindungsgemäße transdermale therapeutische System erlaubt es zudem, eine verbesserte Haftung auf der Haut zu erzielen, da die der Haftung abträglichen Faktoren Feuchtigkeitsstau, Hautquellung und/oder mangelnde Beweglichkeit (Starrheit, mangelnde Elastizität) vermieden oder verringert werden können. Im Vergleich zu einigen Systemen des Stands der Technik kann der Aufwand bei der Konstruktion und Herstellung verringert werden, was die Herstellung vereinfachen und/oder verbilligen kann.

Gleichzeitig ermöglicht das erfindungsgemäße transdermale therapeutische System eine effektive transdermale Verabreichung von Buprenorphin, so dass es beispielsweise zur Schmerztherapie entsprechend Stufe 3 des dreistufigen WHO-Stufenschemas geeignet ist. Dies ist nur möglich, da trotz der fehlenden Okklusion eine hinreichend hohe Menge an Buprenorphin die Haut durchdringt. Dieses Ergebnis war besonders unerwartet, da der Stand der Technik lehrt, dass zur Erzielung einer hinreichenden Hautpermeation von Buprenorphin ein okklusives transdermales therapeutisches System gewählt werden muss und ein nicht-okklusives System ungeeignet wäre.

Die Effizienz des erfindungsgemäßen transdermalen therapeutischen Systems bei der Verabreichung von Buprenorphin kann durch das genannte Merkmal beschrieben werden, dass das erfindungsgemäße transdermale therapeutische System zur Schmerztherapie entsprechend Stufe 3 des dreistufigen WHO-Stufenschemas geeignet ist. Alternativ oder zusätzlich kann die Effizienz des erfindungsgemäßen Systems durch das Merkmal beschrieben werden, dass das transdermale therapeutische System eine Freisetzung einer Gesamtmenge von 1 µg/(cm²*h) oder höher ermöglicht, bezogen auf alle darin vorhandenen Stoffe aus der Gruppe bestehend aus Buprenorphin und seinen Salzen. Eine weitere Möglichkeit, die Effizienz des erfindungsgemäßen Systems alternativ oder zusätzlich zu beschreiben ist, dass das transdermale therapeutische System geeignet ist zur Verabreichung einer bei starken oder stärksten Schmerzen therapeutisch effektiven Menge an Buprenorphin und/oder seinen Salzen. Eine therapeutisch effektive Menge ist hierbei vorzugsweise eine, die eine Blutkonzentration der wirksamen Buprenorphin-Formen von 0,1 ng/ml oder höher ermöglicht, vorzugsweise 0,1 bis 2 ng/ml, insbesondere 0,2 bis 1 ng/ml.

Bevorzugt ist ein erfindungsgemäßes transdermales therapeutisches System, das eine Freisetzung einer Gesamtmenge von 1 µg/(cm²*h) oder höher, besonders bevorzugt 1,2 µg/(cm²*h) oder höher und ganz besonders bevorzugt 1,4 µg/(cm^{2*}h) oder höher ermöglicht, bezogen auf alle darin vorhandenen Stoffe aus der Gruppe bestehend aus Buprenorphin und seinen Salzen. Die Messung kann beispielsweise mit einer Franz-Zelle unter der Verwendung humaner Haut erfolgen (siehe Beispiel 2). Diese freigesetzten Mengen gewährleisten eine effektive Schmerztherapie, insbesondere entsprechend Stufe 3 des dreistufigen WHO-Stufenschemas oder allgemein bei starken und stärksten Schmerzen, insbesondere Tumorschmerzen. Die Freisetzung dieser Mengen erfolgt zumindest am Anfang der Applikation und vorzugsweise über eine Tragedauer von 4 Tagen, bevorzugt 5, 6 oder 7 Tagen und insbesondere noch darüber.

Pharmazeutisch annehmbare Salze von Buprenorphin sind vorzugsweise ausgewählt aus der Gruppe bestehend aus Acetat, Bisulfat, Bitartrat, Citrat, Fumarat, Hydrobromid, Hydrochlorid, Hydroiodid, Lactat, Laurat, Malat, Maleat, Nitrat, Oleat, Palmitat, Phosphat, Stearat, Succinat, Sulfat, Tartrat und Valerat. Ein besonders bevorzugtes Salz ist Hydrochlorid. Besonders bevorzugt ist es, wenn im erfindungsgemäßen System nur Buprenorphin (als Buprenorphin-Base) enthalten ist.

Es ist bevorzugt, dass die Trägerschicht eine der beiden Oberflächenschichten des Systems ist, die Trägerschicht also nur auf einer ihrer beiden Seiten an eine andere Schicht grenzt. Es können zwar zwischen Trägerschicht und Adhäsivschicht eine oder mehrere weitere Schichten vorgesehen sein, vorzugsweise grenzt die Trägerschicht aber unmittelbar an die Adhäsivschicht. Falls vorhanden, ist die ablösbare Schutzschicht eine der beiden Oberflächenschichten des erfindungsgemäßen Systems und befindet sich auf der anderen Seite der Adhäsivschicht als die Trägerschicht. Vorzugsweise grenzt die ablösbare Schutzschicht unmittelbar an die Adhäsivschicht und die Adhäsivschicht ist daran angepasst, auf die Haut aufgebracht zu werden. In manchen Ausführungsformen kann es jedoch vorteilhaft sein, zwischen Adhäsivschicht und ablösbarer Schutzschicht eine weitere Schicht vorzusehen, insbesondere eine wirkstofffreie Kleberschicht (Hautstrich, Unterstrich), die daran angepasst ist, auf die Haut aufgebracht zu werden und in bestimmten Fällen sinnvoll sein kann, um die Haftung an der Haut noch weiter zu verbessern.

Besonders bevorzugt ist ein transdermales therapeutisches System, das die folgende Reihenfolge von aneinander grenzenden Schichten aufweist: Trägerschicht, Adhäsivschicht, ablösbare Schutzschicht, wobei die Trägerschicht vorzugsweise eine Oberflächenschicht ist.

Vorzugsweise ist das erfindungsgemäße transdermale therapeutische System ein Pflaster, insbesondere ein Matrixpflaster. Darunter wird vorliegend ein Pflaster verstanden, in dem der pharmazeutische Wirkstoff in einer Matrix enthalten ist, wobei - anders als bei Membranpflastern - zwischen dem Wirkstoffreservoir und der Haut keine die Abgabe des Wirkstoffs limitierende Lage vorgesehen ist. Weiterhin existiert keine flüssige Reservoirschicht.

Die zur Wirkstoffabgabe ausgebildete Hautkontaktfläche eines transdermalen therapeutischen Systems ist in der Regel direkt proportional zu der Abgaberate (in µg/h) des Wirkstoffs. Der Wirkstoff ist erfindungsgemäß Buprenorphin und/oder eines (oder mehrere) seiner pharmazeutisch annehmbaren Salze. Die Hautkontaktfläche ist die Fläche, die zur Adhäsion auf der Haut bestimmt ist. Unter der zur Wirkstoffabgabe ausgebildeten Hautkontaktfläche ist die Hautkontaktfläche zu verstehen, über die der Wirkstoff an die Haut abgegeben werden kann, vorzugsweise die Fläche, die über eine direkt oder indirekt angrenzende wirkstoffhaltige Schicht (speziell Adhäsivschicht) verfügt. Die Hautkontaktfläche kann größer sein als die zur Wirkstoffabgabe ausgebildete Hautkontaktfläche, da beispielsweise zusätzliche Anteile der Hautkontaktfläche vorgesehen sein können, die nur zur Adhäsion auf der Haut bestimmt sind und nicht zur Wirkstoffabgabe. Ein Beispiel hierfür ist eine wirkstofffreie Kleberschicht, die z.B. die Haut um die zur Wirkstoffabgabe ausgebildete Hautkontaktfläche herum kontaktieren kann.

Aufgrund der hohen Effizienz des erfindungsgemäßen Systems ist es möglich, eine verhältnismäßig kleine zur Wirkstoffabgabe ausgebildete Hautkontaktfläche zu wählen. Eine geringe Hautkontaktfläche ist vorteilhaft hinsichtlich des Tragekomforts und hinsichtlich der Möglichkeit, die Stellen zu variieren, auf denen das System appliziert wird, bewirkt ein nochmals verringertes Restpotential für Hautirritationen und ist vorteilhaft hinsichtlich der Compliance des Patienten. Vorzugsweise beträgt die die Hautkontaktfläche des erfindungsgemäßen transdermalen therapeutischen Systems maximal 50 cm², bevorzugt maximal 40 cm² und insbesondere maximal 30 cm². Die Hautkontaktfläche des erfindungsgemäßen transdermalen therapeutischen Systems kann im Vergleich zu Transtec PRO@ um durchschnittlich 40% reduziert werden. Die Abgaberate des erfindungsgemäßen transdermalen therapeutischen Systems bezogen auf alle darin vorhandenen Stoffe aus der Gruppe bestehend aus Buprenorphin und seinen Salzen kann beispielsweise 35 µg/h, 52,5 µg/h oder 70 µg/h betragen.

Vorzugsweise ist die gesamte Hautkontaktfläche des erfindungsgemäßen transdermalen therapeutischen Systems zur Abgabe von Wirkstoff ausgebildet, d.h. die gesamte Hautkontaktfläche des transdermalen therapeutischen Systems ist eine zur Wirkstoffabgabe ausgebildete Hautkontaktfläche wie oben definiert. Dies ermöglicht einen weiter gesteigerten Tragekomfort und eine aufgrund der Materialeinsparung wirtschaftlichere Herstellung.

Da im Vergleich zu Transtec PRO® keine zusätzliche wirkstofffreie Kleberschicht benötigt wird, um eine identische Haftung auf der Haut zu erzielen, ist es möglich, den Herstellungsprozess einfacher zu gestalten und die Kosten geringer zu halten.

Aufgrund der hohen Effizienz des erfindungsgemäßen transdermalen therapeutischen Systems ist es möglich, eine Beladung mit Buprenorphin zu wählen, die nicht höher ist als diejenige des erwähnten okklusiven Produkts des Stand der Technik Transtec PRO@ (0,8 mg/cm²). Es ist sogar möglich, die Beladung noch weiter zu reduzieren. Bevorzugt ist ein erfindungsgemäßes transdermales therapeutisches System, das einen anfänglichen Gesamtgehalt von 0,8 mg/cm² oder geringer hat, vorzugsweise 0,7 mg/cm² oder geringer und insbesondere 0,6 mg/cm² oder geringer, bezogen auf alle darin vorhandenen Stoffe aus der Gruppe bestehend aus Buprenorphin und seinen Salzen und auf die zur Abgabe von Wirkstoff ausgebildete Hautkontaktfläche. Dies verringert wegen der geringeren benötigten Menge an pharmazeutisch aktiver Substanz die Herstellungskosten. Im Durchschnitt können im Vergleich zu Transtec PRO@ etwa 20% an Wirkstoff eingespart werden. Oftmals kann eine bessere Entleerung des erfindungsgemäßen transdermalen therapeutischen Systems erzielt werden als im Stand der Technik. Wenn im zu entsorgenden verbrauchten System weniger Restwirkstoff enthalten ist, verbessert dies die Sicherheit, da Personen weniger leicht durch unsachgemäße Handhabung in Kontakt mit Buprenorphin kommen und auch die Gefahr des Missbrauchs durch Opiatabhängige verringert wird.

Vorzugsweise ermöglicht das erfindungsgemäße transdermale therapeutische System eine Tragedauer von mindestens oder über 4 Tagen, beispielsweise von 5, 6 oder 7 Tagen oder sogar darüber. Dies kann insbesondere durch eine verbesserte Haftung erreicht werden, beispielsweise dadurch, dass sich keine Feuchtigkeit staut, die den Klebstoff in der Adhäsivschicht beeinträchtigen oder die Haut aufquellen lassen würde, oder dass das erfindungsgemäße System die Bewegungen der Haut gut nachvollziehen kann. Auch können Hautirritationen vermieden werden, die einen vorzeitigen Wechsel der Applikationsstelle nötig machen würden.

Die Wasserdampfdurchlässigkeit des erfindungsgemäßen transdermalen therapeutischen Systems insgesamt und insbesondere der Trägerschicht ist von besonderer Wichtigkeit. Vorzugsweise hat die Trägerschicht eine nach DIN 53122 gemessene Wasserdampfdurchlässigkeit von größer als 400 g/(m²*24 h), bevorzugt von größer als 800 g/(m²*24 h) und insbesondere von größer als 1600 g/(m²*24 h). Materialien mit diesen Werten gewährleisten, dass ein Stau von Wasserdampf besonders gut vermieden wird. Es ist vorteilhaft, wenn nicht nur die Trägerschicht, sondern das gesamte transdermale therapeutische System im zum bestimmungsgemäßen Gebrauch geeigneten Zustand (d.h. ohne die ablösbare Schutzschicht) eine nach DIN 53122 gemessene Wasserdampfdurchlässigkeit von größer als 20 g/(m²*24 h), bevorzugt größer als 60 g/(m²*24 h), besonders bevorzugt größer als 100 g/(m²*24 h), ganz besonders bevorzugt größer als 150 g/(m²*24 h), noch stärker bevorzugt größer als 400 g/(m²*24 h), insbesondere größer als 800 g/(m²*24 h) und am bevorzugtesten von größer als 1600 g/(m²*24 h) aufweist.

Vorteilhaft ist eine nach DIN EN ISO 9237 gemessene Luftdurchlässigkeit der Trägerschicht von größer als 200 l/(m²*s), insbesondere von größer als 400 l/(m²*s), da dann in der Regel der Gasaustausch nicht wesentlich behindert ist und die Haut, auf die das erfindungsgemäße System appliziert ist, gut atmen kann. Vorteilhaft ist auch eine Durchlässigkeit von größer als 200 l/(m²*s), insbesondere von größer als 400 l/(m²*s) für Sauerstoff und/oder Kohlendioxid. Es ist vorteilhaft, wenn nicht nur die Trägerschicht, sondern das gesamte transdermale therapeutische System im zum bestimmungsgemäßen Gebrauch geeigneten Zustand (d.h. ohne die ablösbare Schutzschicht) eine nach DIN EN ISO 9237 gemessene Luftdurchlässigkeit von größer als 200 l/(m²*s), insbesondere von größer als 400 l/(m²*s) aufweist.

Für die Sicherheit des erfindungsgemäßen Systems ist es vorteilhaft, wenn die Trägerschicht im Wesentlichen undurchlässig ist für bestimmte Substanzen und insbesondere für Buprenorphin. Eine Trägerschicht, die den Durchtritt von Flüssigkeiten verhindert, erhöht die Sicherheit des Systems nicht nur dadurch, dass keine Flüssigkeiten aus dem Pflaster in die Umwelt treten können, gegebenenfalls mit darin gelösten Substanzen, sondern auch keine Flüssigkeiten von außen in Richtung Hautoberfläche, was z.B. die Hautquellung und die Permeation unkontrolliert verändern könnte. Es hat sich gezeigt, dass Trägerschichten gut geeignet sind, die gegenüber einem nach DIN 63888 gemessenen hydrostatischen Druck von größer oder gleich 30 mbar, vorzugsweise größer oder gleich 100 mbar und insbesondere größer oder gleich 300 mbar flüssigkeitsundurchlässig sind.

Vorzugsweise stellt die Trägerschicht zusätzlich eine vollständige Barriere für Partikel über 3 µm und bevorzugt eine 99%ige Barriere für Partikel über 0,6 µm dar.

Bevorzugt ist ein erfindungsgemäßes transdermales therapeutisches System, wobei die Trägerschicht gewebtes Textil, nicht-gewebtes Textil oder einen mikroporösen Film umfasst oder daraus besteht. Geeignet als Trägerschicht sind insbesondere Laminate aus mehreren solchen Schichten.

Vorzugsweise umfasst die Trägerschicht Polypropylen, Polyamid, Viscose oder einen Polyester, insbesondere Polyethylenterephthalat (PET).

Für die Trägerschicht besonders geeignete Materialien sind beispielsweise unter der Bezeichnung Securon® erhältlich (Fiberweb Corovin, Peine, Deutschland). Sie bestehen z.B. aus Polypropylen-Trilaminatgewebe (SMS, spunbound-meltblown-spunbound) bzw. aus thermisch bondierten Polypropylen-Endlosfilamenten. Je nach Ausführung zeichnen sich unter anderem durch folgende Eigenschaften aus: a) G47E5S: Flächengewicht 47 g/m² (gemessen nach Edana 40.3-90), Hydrohead (Wassersäule) 510 mm (gemessen nach BBA 030 A 02), Elongation at peak MD (maximale Dehnung "machine direction") 41% (gemessen nach DIN EN ISO 1924-2), Elongation at peak CD ("cross-machine direction") 48% (gemessen nach DIN EN ISO 1924-2), Luftdurchlässigkeit 710 l/(m²*s) (gemessen nach DIN EN ISO 9237), Tensile strength MD (Dehnbarkeit) 28 N (gemessen nach DIN EN ISO 1924-2), Tensile strength CD 15 N (gemessen nach DIN EN ISO 1924-2) und Alcohol repellancy isopropanol (Alkoholabweisung) 95 % (gemessen nach CM280); b) G47HHP: Flächengewicht 47 g/m² (gemessen nach Edana 40.3-90), Tensile strength MD 2,5 kN/m (gemessen nach ISO 1924), Tensile strength CD 1,6 kN/m (gemessen nach ISO 1924), Elongation at peak MD 88% (gemessen nach ISO 1924), Elongation at peak CD 100% (gemessen nach ISO 1924) und Luftdurchlässigkeit 629 l/(m²*s) (gemessen nach DIN EN ISO 9237).

Besonders geeignet für die Trägerschicht ist auch ein Material, das von KOB (Karl Otto Braun KG, Wolfstein, Deutschland) unter der Artikelnummer 053 vertrieben wird. Es handelt sich hierbei um ein bielastisches Gewebe aus PET, das in Längs- und Querrichtung dehnbar ist. Einige seiner Eigenschaften sind wie folgt: Flächengewicht 140 g/m² (ungedehnt) Dehnbarkeit längs 30 +/- 5%, quer größer oder gleich 40%, nach DIN 13726 gemessene Wasserdampfdurchlässigkeit größer als 4400 g/(m²*24 h), Gasdurchlässigkeit 760-960 l/(m²*s) (gemessen nach DIN EN ISO 9237).

In einem bevorzugten erfindungsgemäßen transdermalen therapeutischen System weist die Adhäsivschicht einen Gesamtgehalt zwischen 5 und 20 Gew.-%, vorzugsweise 8-12 Gew.-% an allen darin vorhandenen Stoffen aus der Gruppe bestehend aus Buprenorphin und seinen Salzen auf. Dieser Gehalt hat einen Einfluss auf die Abgaberate (in µg/h) bezogen auf die im System vorhandenen Stoffe aus der Gruppe bestehend aus Buprenorphin und seinen Salzen. Ein hoher Gehalt bedeutet einen geringeren Anteil an anderen Substanzen in der Adhäsivschicht, was durch die geringere Dicke des Systems den Tragekomfort erhöhen kann und durch Materialeinsparung die Wirtschaftlichkeit erhöhen kann.

Vorzugsweise liegen das Buprenorphin und seine Salze in der Adhäsivschicht in einer vollständig gelösten Form vor, d.h. frei von ungelösten Anteilen.

Die Adhäsivschicht umfasst einen druckempfindlichen Klebstoff. Bekannte druckempfindliche Klebstoffe sind beispielsweise Acrylatkleber, Styrolkleber, Silikonkleber, Polyisobutylen, Polyisopren, Polybuten, Polybutadien, Polyvinylacetate, Polyvinylether, Polyvinylalkohol, Polyurethane, Polyester-Elastomere, natürliche Kautschuke, künstliche Kautschuke, Cellulosederivate und Polysaccharide.

Eine Klasse bevorzugter druckempfindlicher Klebstoffe sind Acrylatkleber. Acrylatkleber sind vorzugsweise Polymere, die hergestellt sind unter Verwendung eines oder mehrerer Monomere ausgewählt aus der Gruppe bestehend aus Acrylsäure, Methacrylsäure, Acrylsäureestern (insbesondere Acrylsäurealkylestern), Methacrylsäureestern (insbesondere Methacrylsäurealkylestern) und anderen ungesättigten Monomeren. (Meth)acrylsäurealkylester haben vorzugsweise geradkettige oder verzweigte Alkylgruppen mit 1 bis 12 C-Atomen. Bevorzugte Acrylsäureester sind Methylacrylat, Ethylacrylat, Propylacrylat, Butylacrylat, 2-Ethylhexylacrylat, Glycidylmethacrylat und 2-Hydroxyethylacrylat. Ein bevorzugter Methacrylsäureester ist Methylmethacrylat.

Bevorzugte andere ungesättigte Monomere sind Vinylacetat, Vinylpropionat, Dibutylmaleat, Octylacrylamid, Dimethylacrylamid, Dimethylaminoethylacrylat, Hydroxyethylacrylat, Hydroxypropylacrylat, Methoxyethylacrylat, Methoxyethylmethacrylat und Acrylnitril, insbesondere Vinylacetat. Ein bevorzugter Acrylatkleber ist hergestellt aus einer Kombination von Monomeren, die Acrylsäure, 2-Ethylhexylacrylat und Vinylacetat umfasst. Vorzugsweise ist der Acrylatkleber quervernetzt.

Eine weitere Klasse bevorzugter druckempfindlicher Klebstoffe sind Styrolkleber, insbesondere Styrolkautschuke. Bevorzugt sind Styrol-Butadien-Copolymere oder Styrol-Isopren-Copolymere. Blockcopolymere sind bevorzugt. Die Klebrigkeit der Styrolkleber wurde mit einem geeigneten Haftvermittler (Tackifier) erhöht. Ein besonders gut geeigneter Styrolkleber ist von Henkel Corporation, Bridgewater, New Jersey, USA unter der Bezeichnung DURO-TAK® 87-611A erhältlich. Der Kleber wird als Lösung in organischen Lösungsmitteln zur Verfügung gestellt.

Bevorzugte druckempfindliche Klebstoffe sind auch Silikonkleber. Vorzugsweise sind dies Polysiloxane wie Polydimethylsiloxan und/oder Polymethylphenylsiloxan. Weiterhin bevorzugt wurden diese Kleber durch sogenanntes "End-capping" amin-resistent ausgestattet, wodurch sich das Anwendungssprektrum erhöht. Besonders geeignete Silikonkleber sind von Dow Corning Corporation, Midland, Minnesota, USA unter der Bezeichnung Bio-PSA erhältlich.

Bevorzugt ist ein erfindungsgemäßes transdermales therapeutisches System, wobei die Adhäsivschicht einen Klebstoff enthält ausgewählt aus der Gruppe bestehend aus Acrylatklebern, Acrylatklebern mit Vinylacetat-Anteil, Styrol klebern und Silikonklebern. Besonders bevorzugt ist eine Adhäsivschicht, die ein Acrylat-Copolymerisat enthält, insbesondere ein Acrylat-Vinylacetat-Copolymerisat. Besonders gut geeignete AcrylatCopolymerisate und Acrylat-Vinylacetat-Copolymerisate werden unter der Bezeichnung DURO-TAK® angeboten (Henkel Corporation, Bridgewater, New Jersey, USA), beispielsweise DURO-TAK® 87-2825 oder DURO-TAK® 387-2825.

Die genannten Klebstoffe lassen sich nicht nur für die Adhäsivschicht einsetzen, sondern auch für die gegebenenfalls vorhandene zusätzliche wirkstofffreie Kleberschicht, bei der Silikonkleber besonders bevorzugt sind (z.B. BioPSA 7-4301 erhältlich von Dow Corning Corporation, Midland, Minnesota, USA mit 1% Silikonöl 1000,).

Durch Wahl von geeigneten Schichtdicken lassen sich Systeme herstellen, die vorteilhafte Eigenschaften hinsichtlich Effizienz, Tragekomfort, Tragedauer und Wirtschaftlichkeit haben. Mit der Schichtdicke in Zusammenhang steht das Flächengewicht (in g/m²). Bevorzugt ist ein erfindungsgemäßes transdermales therapeutisches System, wobei die Trägerschicht ein Flächengewicht von 30-160 g/m², die Adhäsivschicht ein Flächengewicht von 40-80 g/m² und/oder die ablösbare Schutzschicht ein Flächengewicht von 80 bis 200 g/m² aufweist, jeweils gemessen nach ISO 536. Die gegebenenfalls vorhandene zusätzliche wirkstofffreie Kleberschicht hat vorzugsweise ein Flächengewicht von 10 bis 40 g/m².

Insgesamt sind Schichtdicken bevorzugt, die bei 20 µm oder darüber liegen. Bevorzugte Schichtdicken der Adhäsivschicht sind 15 bis 140 µm, besonders bevorzugt 30 bis 100 µm und ganz besonders bevorzugt 40 bis 80 µm.

Die ablösbare Schutzschicht basiert vorzugsweise auf einem Material wie Polyester (z.B. Polyethylenterephthalat), Polypropylen, Polyvinylchlorid, Polyethylen hoher Dichte (HDPE), Aluminium oder Papier, das für Buprenorphin undurchlässig sind und leicht von der Adhäsivschicht ablösbar ist, entweder von sich aus oder durch Behandlung mit Silikon, Fluorsilikon, Fluorcarbon (z.B. Polytetrafluorethylen) oder Polyethylen. Vorteilhafte Materialien sind z.B. silikonisiertes PET und Fluorpolymer-beschichtetes PET. Geeignete ablösbare Schutzschichten werden beispielsweise vertrieben von Loparex (Apeldoorn, Niederlande) unter den Bezeichnungen Primeliner® FL2000 PET 100µm 1S oder Primeliner® SN 410/140 oder von 3M (St. Paul, Minnesota, USA) unter der Bezeichnung Scotchpak 1022. Die Dicke der ablösbaren Schutzschicht kann beispielsweise 80 bis 120 µm betragen, gemessen nach ISO 534.

Das erfindungsgemäße transdermale therapeutische System enthält optional weitere Komponenten. Eine bevorzugte weitere Komponente sind Hautpermeationsförderer, vorzugsweise Glycole, Öle und Fette, Harnstoffderivate, Salicylsäure, Benzylnicotinat, gesättigte oder ungesättigte Fettalkohole mit 8 bis 18 C-Atomen oder Ester davon, gesättigte oder ungesättigte Fettsäuren mit 8 bis 18 C-Atomen oder Ester davon, Monoglyceride, Diglyceride, Triglyceride, Diethanolamide, N,N-Dimethylamide, Ölsäure, Propyloleat, Oleylacetat, Propylmyristat, Myristylalkohol, Stearinsäure, Stearylalkohol, Stearylpropylester, Myristyl-N,N-Dimethylamid, Caprylalkohol, Ethylcaprylat, Hexamethylenlauramid, Natriumlaurylsulfat und/oder Hexamethylenpalmitat. Hautpermeationsförderer liegen vorzugsweise in der Adhäsivschicht vor.

Andere bevorzugte weitere Komponenten sind weitere Wirkstoffe, Verdickungsmittel, Weichmacher, Emulgatoren und/oder Stabilisatoren.

In einer bevorzugten Ausführungsform ist das erfindungsgemäße TTS jedoch frei von diesen weiteren Komponenten, d. h. es enthält keine Permeationsverstärker, Verdickungsmittel, Weichmacher oder Emulgatoren.

Bevorzugt ist ein erfindungsgemäßes transdermales therapeutisches System, das frei ist von Aceton und das eine ablösbare Schutzschicht und, bezogen auf die zur Abgabe von Wirkstoff ausgebildete Hautkontaktfläche, 0,36-0,8 mg/cm² Buprenorphin-Base und 4,4-5,2 mg/cm² Acrylatkleber umfasst. Vorzugsweise ist die zur Abgabe von Wirkstoff ausgebildete Hautkontaktfläche 25 cm². Wenn bei der Herstellung Aceton verwendet wird, so wird dieses, um die Acetonfreiheit zu gewährleisten, vorzugsweise während der Trocknung entfernt.

Gemäß einem weiteren Aspekt betrifft die vorliegende Erfindung ein Verfahren zur Herstellung eines erfindungsgemäßen transdermalen therapeutischen Systems mit den Schritten Herstellen einer flüssigen Adhäsivzusammensetzung aus (i) Buprenorphin und/oder einem seiner pharmazeutisch annehmbaren Salze, (ii) einem Klebstoff und (iii) einer geeigneten Flüssigkeit, Herstellen eines Mehrschichtsystems durch Beschichten einer ersten Schicht mit der flüssigen Adhäsivzusammensetzung, Trocknen des Mehrschichtsystems, optional Hinzufügen einer weiteren Schicht zum Mehrschichtsystem und Zerteilen des Mehrschichtsystems in kleinere Flächeneinheiten.

Vorzugsweise ist die zu beschichtende erste Schicht die Trägerschicht. Das optionale Hinzufügen einer weiteren Schicht ist in diesem Fall die Applikation einer ablösbaren Schutzschicht.

Alternativ kann die zunächst die Schutzschicht mit der flüssigen Adhäsivzusammensetzung beschichtet werden. In diesem Fall wird als weitere Schicht zum Mehrschichtsystem die Trägerschicht hinzugefügt.

Vorzugsweise erfolgt das Zerteilen durch Ausstanzen.

Ein weiterer Aspekt der vorliegenden Erfindung betrifft die Verwendung einer nicht-okklusiven Schicht als Trägerschicht für ein erfindungsgemäßes Buprenorphin und/oder eines seiner pharmazeutisch annehmbaren Salze enthaltendes transdermales therapeutisches System.

### Beispiele:

### Beispiel 1: Herstellung eines erfindungsgemäßen transdermalen therapeutischen Systems

Es wurde ein möglichst einfaches Herstellverfahren angestrebt.

### a) Herstellung einer flüssigen Adhäsivzusammensetzung aus Buprenorphin, einem Klebstoff und einer geeigneten Flüssigkeit

Buprenorphin-Base wird abgewogen und in Aceton dispergiert. Die Zusammensetzung wird gemischt. Es bildet sich eine homogene, weiße, blasenfreie Dispersion (Sichtprüfung). Der Klebstoff (z.B. DURO-TAK® 387-2825) wird abgewogen und hinzugefügt. Nach Mischen bildet sich eine Lösung, die nach Sichtprüfung homogen, klar, frei von ungelösten Partikeln und frei von Blasen ist.

### b) Herstellen eines Mehrschichtsystems

Eine silikonisierte Schutzschicht (z.B. Primerliner® PET 100 µm) wird mit der flüssigen Adhäsivzusammensetzung mittels eines Rakels in einer halbkontinuierlichen Benchtop-Beschichtungsapparatur beschichtet. Die Beschichtungsparameter wie z.B. die Geschwindigkeit und Beschichtungsdicke werden kontinuierlich überprüft.

### c) Trocknen des Mehrschichtsystems

Die Lösungsmittel werden durch Halten bei 70°C für 15 min und 100°C für 5 min in einem Trocknungsofen entfernt. Die Temperatur wird kontinuierlich überprüft.

### d) Hinzufügen einer weiteren Schicht zum Mehrschichtsystem

Die Trägerschicht (z.B. KOB Nr. 053, weiß) wird in geeignete Stücke geschnitten und auf die getrocknete Matrix laminiert. Die Matrix ist homogen und klar, ohne Blasen oder Partikel. Das Flächengewicht ist 49,5 bis 60,5 g/m².

### e) Zerteilen des Mehrschichtsystems in kleinere Flächeneinheiten

Fertige transdermale therapeutische Systeme in einer Größe von 25 cm² werden durch Stanzen aus dem Mehrschichtsystem erhalten. Das Stanzwerkzeug wird auf ein geeignetes Stück des Mehrschichtsystems mit hinreichendem Abstand von den Kanten aufgesetzt. Pflaster werden dann ausgeschnitten, indem mittels einer handbetriebenen Presse Druck auf das Stanzwerkzeug ausgeübt wird. Die Pflaster haben ohne Schutzschicht ein Gewicht von 438,8 bis 536,3 mg.

### f) Verpacken

Taschen aus einem Kompositfolienmaterial werden hergestellt, indem drei Seiten miteinander verschweißt werden und eine Seite offen gelassen wird. Die einzelnen transdermalen therapeutischen Systeme werden getrennt voneinander in die Taschen eingeführt. Die vierte Seite der Tasche wird danach verschweißt.

### g) Etikettieren

Die Taschen werden mit kommerziell erhältlichen papierbasierten selbstklebenden Etiketten versehen.

### Beispiel 2: Franz-Zelle

Permeationsexperimente durch (humane) Haut oder künstliche Membranen stellen ein wichtiges Instrument zur Bewertung von Pflastern dar. Zur Untersuchung der Permeation wird Haut oder eine künstliche Membran zwischen zwei Kompartimente gespannt. Dadurch entstehen zwei Bereiche, ein Donor- und ein Akzeptorkompartiment. Im Donorkompartiment kann eine wirkstoffhaltige Flüssigkeit eingefüllt oder ein Pflaster mit Wirkstoff auf die Trennmembran (Haut) aufgeklebt werden. Das Akzeptorkompartiment wird mit einer Aufnahmeflüssigkeit versehen. Diese sollte physiologisch verträglich sein und ausreichende Löslichkeit für den Wirkstoff aufweisen. Mit diesem System kann die Diffusion vom Donor in den Akzeptor über die Zeit beobachtet werden.

Vorliegend wurden sog. "Franz-Zellen" verwendet, die die Möglichkeit bieten, Tier- oder Humanhaut einzuspannen, und damit die Barriereeigenschaften der Haut simulieren. Mit Hilfe der Franz-Zellen lassen sich Voraussagen zur Diffusion von Stoffen durch die Haut treffen. Es werden hier die Franz-Zellen modifiziert nach Chien verwendet.

In die Franz-Zelle wurde dermatomisierte Humanhaut (500 µm) eingespannt und Pflasterproben auf die Haut aufgeklebt. Die Mustergröße betrug 1,5 cm². Das Rezeptorkompartiment wurde mit 40 ml Phosphatpuffer pH 3,00 Ph.Eur. + 0,1% Natriumazid befüllt. Die Franz-Zellen wurden verschlossen und im Wasserbad auf 32°C temperiert. Zu vorgegebenen Zeitpunkten wurde eine Probe von 2 ml aus dem Rezeptorkompartiment gezogen und mittels HPLC analysiert. Entnommene Volumina wurden mit dem gleichen Volumen frischer Pufferlösung ersetzt.

In der Franz-Zelle kann die Abgabe von Wasserdampf durch die Haut und somit die Okklusion simuliert werden, indem die Zelle entsprechend temperiert wird und die eingespannte Haut als Barriere wirkt.

### Beispiel 3: Hautpermeationsstudien

Transdermale therapeutische Systeme mit folgenden Adhäsivschichten wurden gemäß Beispiel 1 hergestellt:
- Acrylatkleber (Acrylat-Vinylacetat)
Hergestellt werden, als Beispiele für bevorzugte transdermale therapeutische Systeme mit 25 cm² zur Abgabe von Wirkstoff ausgebildeter Hautkontaktfläche, die 9 - 20 mg Buprenorphin-Base, 110 - 130 mg Acrylatkleber (z.B. DURO-TAK® 87-2825) und eine ablösbare Schutzschicht (z.B. eine silikonisierte PET-Folie wie Primeliner® FL2000) enthalten und die frei sind von Aceton:

| **Komponente** | Beispiel 3A | Beispiel 3B | Beispiel 3C |
|---|---|---|---|
| Buprenorphin-Base | 15 mg | 12,4 | 20,0 mg |
| DURO-TAK® 87-2825 | 122,5 mg | 125,2 | 118,0 mg |
| Aceton | - (während Trocknung entfernt) | - (während Trocknung entfernt) | - (während Trocknung entfernt) |
| PET bielastisches Gewebe (z.B. KOB Nr. 053) | Quantum satis | Quantum satis | Quantum satis |
| Silikonisierte PET-Folie (z.B. Primeliner® FL2000) | Quantum satis | Quantum satis | Quantum satis |

| **Komponente** | Beispiel 3D | Beispiel 3E | |
|---|---|---|---|
| Buprenorphin-Base | 15 mg | 20 mg 20 mg | |
| DURO-TAK® 87-2825 | 122,5 mg | 118,0 mg 118,0 mg | |
| Aceton | - (während Trocknung entfernt) | - (während Trocknung entfernt) | |
| Spunlaid PP Vlies (z.B. Securon Classic) | Quantum satis | - | |
| Bielastisches Polyamid-Gewebe (z.B. Cordura bielastisch) | - | Quantum satis | |

- Silikonkleber

| **Komponente** | Beispiel 3F |
|---|---|
| Buprenorphin-Base | 20,0 mg |
| BioPSA 7-4301 | 60,0 |
| BioPSA 7-4201 | 60,0 |
| Kollidon 90F | 5,84 |
| Aceton | - (während Trocknung entfernt) |
| Ethanol absolut | - (während Trocknung entfernt) |
| Silikonisierte PET-Folie (z.B. Primeliner® FL2000) | Quantum satis |
| PET bielastisches Gewebe (z.B. KOB Nr. 053) | Quantum satis |

- Styrolkleber

| **Komponente** | Beispiel 3G | Beispiel 3H | Beispiel 3l |
|---|---|---|---|
| Buprenorphin-Base | 10,0 mg | 16,3 mg | 20 mg |
| DURO-TAK® 87-611A | 125,0 mg | 118,8 mg | 115,0 mg |
| BioPSA 7-4301 | - | 49,5 mg | - |
| Silikonöl 1000 | - | 0,5 mg | - |
| Aceton | - (während Trocknung entfernt) | - (während Trocknung entfernt) | - (während Trocknung entfernt) |
| PET bielastisches Gewebe (z.B. KOB Nr. 053) | Quantum satis | Quantum satis | Quantum satis |
| Silikonisierte PET-Folie (z.B. Primeliner® FL2000) | Quantum satis | - | Quantum satis |
| Fluorpolymerbeschichtete PET-Folie (z.B. Scotchpak 1022) | - | Quantum satis | - |

Unter Verwendung von Silikonkleber war die Adhäsivschicht ein disperses System mit Mikrotröpfchen. Bei den anderen Systemen war das Buprenorphin vollständig in der Matrix gelöst.

Die verschiedenen erfindungsgemäßen Systeme wurden in Franz-Zell-Hautpermeationsexperimenten (siehe Beispiel 2) über einen Zeitraum von bis zu 96 Stunden auf Ihre Abgabeeigenschaften für Buprenorphin geprüft. Ein Transtec PRO®-Pflaster (okklusiv) diente jeweils als Referenz.

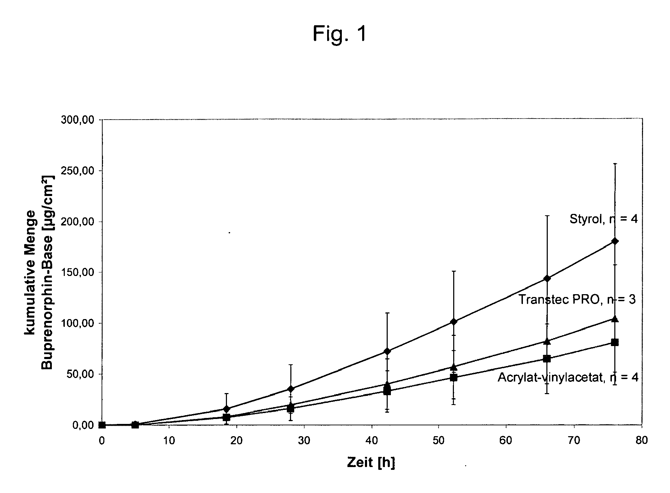

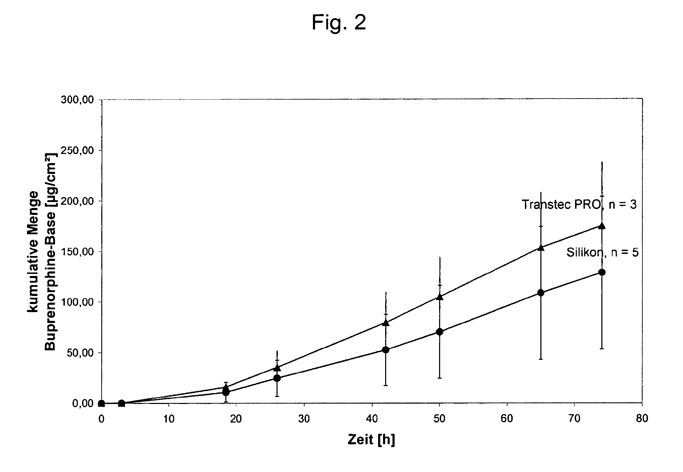

Alle erfindungsgemäßen Systeme gemäß Fig. 1 und 2 hatten eine nicht-okklusive Trägerschicht als Oberflächenschicht und einen Buprenorphin-Base-Gehalt von 0,8 mg/cm².

Die Ergebnisse sind in Fig. 1 bis 3 dargestellt. Gezeigt ist jeweils die kumulierte Permeation von Buprenorphin durch die Haut.

In den Experimenten zeigte sich, dass sich Styrol kleber, Acrylat-Vinylacetat-Kleber und Silikonkleber gut als Adhäsivschicht und Trägermatrix für Buprenorphin eignen. Die Buprenorphin-Beladung konnte in weiteren Versuchen sogar noch verringert werden.

Fig. 1 zeigt, dass das erfindungsgemäße System mit Adhäsivschicht basierend auf Styrolkleber (rautenförmige Symbole, 4 Messungen, entspricht Beispiel 3l) eine tendenziell noch bessere Permeation von Buprenorphin durch die Haut bewirkt als Transtec PRO@ (dreieckige Symbole, 3 Messungen). Ein erstes System mit einer Adhäsivschicht basierend auf Acrylat-Vinylacetat (quadratische Symbole, 4 Messungen, entspricht Beispiel 3C) als Adhäsivschicht war im Rahmen der Messgenauigkeit nicht unterscheidbar von Transtec PRO®. Alle Systeme hatten eine Beladung von 0,8 mg/cm².

Fig. 2 zeigt, dass ein System mit Adhäsivschicht basierend auf Silikonkleber (runde Symbole, 5 Messungen) hinsichtlich der Buprenorphin-Permeation im Rahmen der Messgenauigkeit nicht unterscheidbar von Transtec PRO@ (dreieckige Symbole, 3 Messungen) ist. Alle Systeme hatten eine Beladung von 0,8 mg/cm².

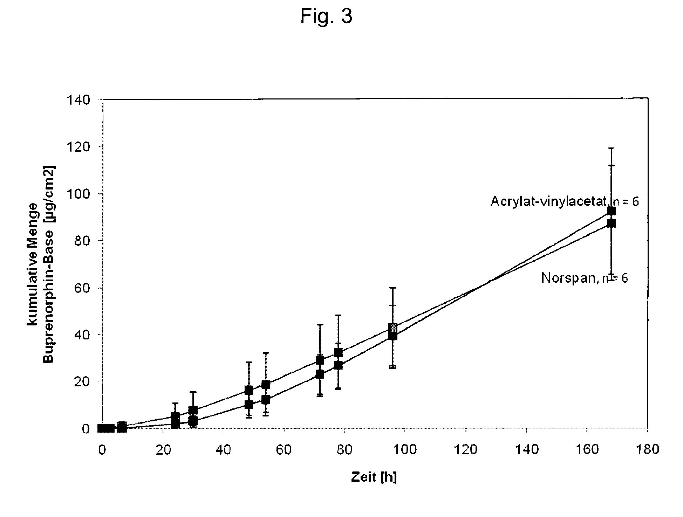

In Fig. 3 ist der Vergleich von Norspan® (anfänglicher Gesamtgehalt an Buprenorphin von 0,8 mg/cm², 7-Tages-Produkt) mit einem erfindungsgemäßen nicht-okklusiven transdermalen therapeutischen System mit Acrylatkleber (entspricht Beispiel 3A) gezeigt, Der anfängliche Gesamtgehalt an Buprenorphin betrug 0,6 mg/cm². Trotz non-okklusiver Bedingungen und im Vergleich niedrigerer Gesamtbeladung an Buprenorphin-Base konnten über 7 Tage äquivalente Flux-Werte erzielt werden.

Im Ergebnis gewährleistete das erfindungsgemäße nicht-okklusive System im Rahmen der Messgenauigkeit eine identische Permeation von Buprenorphin durch die Haut wie die okklusiven Systeme Transtec PRO@ und Norspan®.

### Beispiel 4: Lagerstabilität

Die Lagerstabilität eines erfindungsgemäßen transdermalen therapeutischen Systems (anfänglicher Gesamtgehalt an Buprenorphin 0,6 mg/cm², Trägerschicht KOB Art. 053, Adhäsivschicht Acrylat-vinylacetat mit 10,91 Gew.-% Buprenorphin) wurde mittels HPLC-Analyse getestet.

Die Analyse von Verunreinigungen erfolgte bei Beginn der Messungen und nach einer Lagerung bei 40°C und 75% relativer Luftfeuchte nach 1 Monat. Es wurden keine wesentlichen Änderungen festgestellt. Es wurden drei Peaks von Verunreinigungen festgestellt, die gemeinsam einen Anteil von 0,37% des deklarierten Gehalts hatten.

Der Wirkstoffgehalt unterschied sich nach 0 und 1-3 Monaten nicht.

Weitere Daten zum Verhalten von Buprenorphin-Base im Acrylatklebersystem wurden durch eine Vorcharge erhalten, die nach 1 und 3 Monaten 40°C/75% rh-Lagerung vergleichbare Werte zeigten. Die Summe der Verunreinigungen betrug hier 0,45% und 0,65% nach 1 bzw. 3 Monaten.
Der Gehalt blieb bei der Vorcharge konstant im Spezifikationsfenster von 90-110%. Die Vorcharge unterschied sich im verwendeten Trägermaterial von der Charge des erfindungsgemäßen Systems.

### Beispiel 5: Transdermales therapeutisches System

Das nachfolgend beschriebene besonders bevorzugte erfindungsgemäße TTS hat eine vollständig zur Abgabe von Wirkstoff ausgebildete Hautkontaktfläche von 25 cm²:

| **Adhäsivschicht** | | | |
|---|---|---|---|
| Buprenorphin-Base | 15 mg | (Wirkstoff) | Standard: EP |
| | | | |
| DURO-TAK® 87-2825 | 122,5 mg | (Klebstoff) | Standard: hausintern |
| Aceton | - (während der Beschichtung entfernt) | | Standard: EP |

| **Weitere Schichten** | | | |
|---|---|---|---|
| PET bielastisches Gewebe (KOB Nr. 053) | | (Trägerschicht) | Standard: hausintern |
| Silikonisierte PET-Folie (Primeliner® FL2000) | | (Ablösbare Schutzschicht) | Standard: hausintern |

Das transdermale therapeutische System wird in Papier/Aluminium/Polyethylen-Kompositmaterial verpackt.

## Patentansprüche

1. Transdermales therapeutisches System umfassend
- eine Trägerschicht,
- eine Adhäsivschicht, die Buprenorphin und/oder eines seiner pharmazeutisch annehmbaren Salze enthält,
- und optional eine ablösbare Schutzschicht,
wobei das transdermale therapeutische System nicht-okklusiv ist und zur Schmerztherapie entsprechend Stufe 3 des dreistufigen WHO-Stufenschemas geeignet ist.

2. Transdermales therapeutisches System nach Anspruch 1, wobei das transdermale therapeutische System eine Freisetzung einer Gesamtmenge von 1 µg/(cm²*h) oder höher ermöglicht, bezogen auf alle darin vorhandenen Stoffe aus der Gruppe bestehend aus Buprenorphin und seinen Salzen.

3. Transdermales therapeutisches System nach einem der vorigen Ansprüche, wobei das transdermale therapeutische System einen anfänglichen Gesamtgehalt von 0,8 mg/cm² oder geringer hat, bezogen auf alle darin vorhandenen Stoffe aus der Gruppe bestehend aus Buprenorphin und seinen Salzen und auf die zur Abgabe von Wirkstoff ausgebildete Hautkontaktfläche.

4. Transdermales therapeutisches System nach einem der vorigen Ansprüche, wobei die Hautkontaktfläche des transdermalen therapeutischen Systems maximal 50 cm² beträgt.

5. Transdermales therapeutisches System nach einem der vorigen Ansprüche, wobei die gesamte Hautkontaktfläche des transdermalen therapeutischen Systems zur Abgabe von Wirkstoff ausgebildet ist.

6. Transdermales therapeutisches System nach einem der vorigen Ansprüche, wobei das transdermale therapeutische System eine Tragedauer von mindestens, bevorzugt über, 4 Tagen ermöglicht.

7. Transdermales therapeutisches System nach einem der vorigen Ansprüche, wobei die Trägerschicht eine nach DIN 53122 gemessene Wasserdampfdurchlässigkeit von größer als 800 g/(m²*24 h) und eine nach DIN EN ISO 9237 gemessene Luftdurchlässigkeit von größer als 200 l/(m²*s) aufweist und gegenüber einem nach DIN 63888 gemessenen hydrostatischen Druck von größer oder gleich 30 mbar flüssigkeitsundurchlässig ist.

8. Transdermales therapeutisches System nach einem der vorigen Ansprüche, wobei die Trägerschicht gewebtes Textil, nicht-gewebtes Textil oder einen mikroporösen Film umfasst oder daraus besteht.

9. Transdermales therapeutisches System nach einem der vorigen Ansprüche, wobei die Trägerschicht Polypropylen, Polyamid, Viscose oder einen Polyester umfasst.

10. Transdermales therapeutisches System nach einem der vorigen Ansprüche, wobei die Adhäsivschicht einen Gesamtgehalt zwischen 5 und 20 Gew.-% an allen darin vorhandenen Stoffen aus der Gruppe bestehend aus Buprenorphin und seinen Salzen aufweist.

11. Transdermales therapeutisches System nach einem der vorigen Ansprüche, wobei die Adhäsivschicht einen Klebstoff enthält ausgewählt aus der Gruppe bestehend aus Acrylatklebern, Acrylatklebern mit Vinylacetat-Anteil, Styrolklebern und Silikonklebern.

12. Transdermales therapeutisches System nach einem der vorigen Ansprüche, wobei die Trägerschicht ein Flächengewicht von 30-160 g/m² und/oder die Adhäsivschicht ein Flächengewicht von 40-80 g/m² aufweist, jeweils gemessen nach ISO 536.

13. Transdermales therapeutisches System nach einem der vorigen Ansprüche, das frei ist von Aceton und das eine ablösbare Schutzschicht und, bezogen auf die zur Abgabe von Wirkstoff ausgebildete Hautkontaktfläche, 0,36-0,8 mg/cm² Buprenorphin-Base und 4,4-5,2 mg/cm² Acrylatkleber umfasst.

14. Verfahren zur Herstellung eines transdermalen therapeutischen Systems nach einem der vorigen Ansprüche mit den Schritten
- Herstellen einer flüssigen Adhäsivzusammensetzung aus (i) Buprenorphin und/oder einem seiner pharmazeutisch annehmbaren Salze, (ii) einem Klebstoff und (iii) einer geeigneten Flüssigkeit,
- Herstellen eines Mehrschichtsystems durch Beschichten einer ersten Schicht mit der flüssigen Adhäsivzusammensetzung,
- Trocknen des Mehrschichtsystems,
- optional Hinzufügen einer weiteren Schicht zum Mehrschichtsystem und
- Zerteilen des Mehrschichtsystems in kleinere Flächeneinheiten.

15. Verwendung einer nicht-okklusiven Schicht als Trägerschicht für ein Buprenorphin und/oder eines seiner pharmazeutisch annehmbaren Salze enthaltendes transdermales therapeutisches System nach Anspruch 1.
